# EUROPÄISCHE PATENTSCHRIFT

(11) **EP 3 021 875 B1**
(45) Veröffentlichungstag und Bekanntmachung des Hinweises auf die Patenterteilung: **18.09.2019**
(21) Anmeldenummer: 14752949.9
(22) Anmeldetag: 07.07.2014
(51) Int. Cl.: A61L 2/14, H05H 1/24

(54) **ANORDNUNG ZUR KEIMREDUKTION MITTELS PLASMA**
SYSTEM FOR REDUCING GERMS BY MEANS OF PLASMA
SYSTÈME DE RÉDUCTION DES GERMES PAR PLASMA

(30) Priorität: 15.07.2013 DE 102013107448
(43) Veröffentlichungstag der Anmeldung: 25.05.2016
(73) Patentinhaber: Relyon Plasma GmbH, 93055 Regensburg (DE)
(72) Erfinder: NETTESHEIM, Stefan, 93051 Regensburg (DE)
(74) Vertreter: Reichert & Lindner Partnerschaft Patentanwälte
(86) Internationale Anmeldenummer: PCT/IB2014/062922
(87) Internationale Veröffentlichungsnummer: WO 2015/008191

(56) Entgegenhaltungen:
- EP-A1- 2 170 022
- WO-A1-2011/055113
- DE-U1-202008 008 733
- DE-U1-202008 008 980

## Beschreibung

Die Erfindung betrifft eine Anordnung zur Keimreduktion mittels Plasma gemäß Anspruch 1.

Ferner betrifft die vorliegende Erfindung die Verwendung dieser Anordnung zur Keimreduktion bzw. Keimabtötung gemäß Anspruch 12.

Bevorzugte Ausführungsformen der vorliegenden Erfindung werden in den abhängigen Ansprüchen definiert.

Aus der deutschen Patentschrift DE 10 2008 018 827 B4 ist eine Vorrichtung zur Erzeugung eines Plasmas mit einem Piezoelement offenbart. Das Piezoelement ist mit einem Primär- und Sekundärbereich ausgestattet. Dabei erfolgt die Ansteuerung des Primärbereichs des Piezoelements mit Niederspannung und Hochfrequenz. In der Folge wird ein Plasma durch die Feldüberhöhung auf der Fläche des Sekundärbereichs des Piezoelements gezündet.

Die deutsche Patentanmeldung DE 10 2007 054 161 A1 offenbart ein Verfahren zur Sterilisation von gestreckten Werkstücken. Mit dem hier beschriebenen Plasmabehandlungsverfahren wird eine Oberflächendekontamination erreicht, um Mikroorganismen und Viren mit einem Niedertemperaturplasma abzutöten. Das Verfahren zeichnet sich dadurch aus, dass dem Plasma verschiedene geeignete Zusätze beigemischt werden, um eine möglichst gute Abtötung der Mikroorganismen, bzw. Viren zu erreichen. Das Verfahren wird bei nicht organischen Körpern angewendet.

Die chinesische Patentanmeldung CN 101259036 A offenbart einen Mikroplasma-Stift zum Entfernen von Sommersprossen. Der Stift umfasst einen Plasmakopf zur Hautreinigung, ein Griffgehäuse, einen Mikro-Wandler, der im Griffgehäuse angeordnet ist, einen Leistungskontroller und ein Leistungsmodul. Der Stift kann eine hohe und variable Plasmaleistung abgeben, wofür eine integrierte Schaltung oder ein Halbleiter-Chip-Mikroprozessor im Griffgehäuse installiert ist, die einen piezoelektrischen Transformator treibt.

Die internationale Patentanmeldung WO 2010/034451 A1 offenbart einen Plasma-Applikator zum Anlegen eines nicht-thermischen Plasmas auf eine Oberfläche. Die Vorrichtung dient insbesondere für die Plasmabehandlung von lebendem Gewebe und insbesondere für die Plasmabehandlung von Wunden.

Die deutsche Patentanmeldung DE 10 2009 028 190 A1 offenbart eine Vorrichtung zum Erzeugen eines nicht-thermischen Atmosphärendruck-Plasmas. In einem Metallgehäuse, das als geerdete Elektrode fungiert, ist ein HF-Generator, eine HF-Resonanzspule mit einem, für die Hochfrequenz geeigneten geschlossenen Ferritkern, ein als Gasdüse fungierender Isolierkörper, sowie eine in dem Isolierkörper gehalterte Hochspannungselektrode in der Weise angeordnet, dass sie vom Prozessgas um- beziehungsweise durchströmt werden.

Die deutsche Patentanmeldung DE 10 2011 001 416 A1 offenbart eine Plasmabehandlungseinrichtung zur Behandlung von Wunden oder erkrankten Hautpartien. Die Plasmabehandlungseinrichtung weist zwei flexible Flächenelektroden zur Erzeugung eines nicht-thermischen Plasmas auf. Die beiden Flächenelektroden bestehen jeweils aus mindestens einem elektrischen Leiter, wobei die Leiter miteinander verwoben sind. Auf der der zu behandelnden Oberfläche zugewandten Außenseite der Flächenelektroden ist eine Wundkontaktschicht aus einem antiseptisch behandelten Material lösbar befestigt.

Die internationale Patentanmeldung WO 2010/022871 A1 offenbart eine Vorrichtung zur Wundbehandlung mittels eines nicht-thermischen Plasmas. Das Plasma umfasst ein teilweise ionisiertes Trägergas und mindestens ein Additiv, das vorzugsweise einen sterilisierenden Effekt hat, der die Wundheilung verbessert.

Die internationale Patentanmeldung WO 2010/034451 A1 offenbart einen Plasma-Applikator zum Anlegen eines nicht-thermischen Plasmas auf eine Oberfläche, insbesondere für die Plasmabehandlung von lebendem Gewebe und insbesondere für die Plasmabehandlung von Wunden. Der Plasma-Applikator umfasst einen Verschlussdeckel zum Abdecken eines Teils der Oberfläche. Dadurch wird ein Hohlraum zwischen dem Verschlussdeckel und der Oberfläche geschaffen. Das nicht-thermische Plasma ist im Hohlraum vorgesehen und zusätzlich kann der Hohlraum mit Gas gespült werden. Ebenso ist eine Pumpe vorgesehen, die Gas aus dem Hohlraum absaugt.

Die internationale Patentanmeldung WO 2011/023478 A1 offenbart eine Vorrichtung zur flächigen Behandlung von Bereichen menschlicher oder tierischer Haut mittels eines kalten Plasmas unter atmosphärischem Druck. Hierzu ist eine dielektrische behinderte Entladung vorgesehen, die auf die Oberfläche einwirkt. Die Vorrichtung umfasst mindestens ein flexibles Isoliermaterial, eine flexible Hochspannungselektrode, ein flexibles Dielektrikum, eine flexible geerdete Elektrode und eine Gaszufuhr. Die flexible Hochspannungselektrode ist in dem isolierenden Elastomer eingebettet.

Die internationale Patentanmeldung WO 2011/110191 A1 offenbart eine Anordnung zur Behandlung eines flächigen Objekts mit einem Niedertemperatur-Plasma, insbesondere zum Sterilisieren des Objekts. Das Niedertemperatur-Plasma wird auf eine Oberfläche des Gegenstands aufgebracht, wobei das Niedertemperatur-Plasma durch einen Umschlag angelegt wird, so dass die Niedertemperatur-Plasmabehandlung durch die Hülle eindringt.

Die internationale Patentanmeldung WO 2012/158443 A2 offenbart eine Vorrichtung zur Erzeugung eines kalten Plasmas. Die Vorrichtung umfasst eine von Hand gehaltene Düse mittels der das Plasma zur Heilung von Wunden, zur Verbesserung von Anomalien der Hauoberfläche und zum Abtöten von Krankheitserregern auf die zu behandelnde Stelle gerichtet wird.

Das deutsche Gebrauchsmuster DE 20 2008 008 980 U1 offenbart eine Vorrichtung zur Erzeugung eines Atmosphärendruck-Plasmas. Die Vorrichtung weist ein Gehäuse mit einer dielektrischen Endkappe auf. Innerhalb des Gehäuses ist im Bereich der Endkappe, ein sich in Längsrichtung des Gehäuses erstreckendes Piezoelement angeordnet, wobei eine stirnseitige Austrittsöffnung des Gehäuses von der Endkappe gebildet wird. Am Piezoelement kann zur Erzeugung eines Plasmas eine Wechselspannung angelegt werden. Innerhalb des Gehäuses ist eine Ansteuerplatine für das Piezoelement vorgesehen.

Das deutsche Gebrauchsmuster DE 20 2008 008 733 U1 offenbart eine Vorrichtung zur Behandlung von Gegenständen. Die zu behandelnden Gegenstände werden in einen Behälter aufgenommen. Es ist mindestens eine innere Elektrode an der Innenseite des Behälters und eine mit der mindestens einen inneren Elektrode zusammenwirkende äußere Elektrode vorgesehen. Die äußere Elektrode ist an eine Spannungsversorgung angeschlossen. Zwischen der äußeren Elektrode und der inneren Elektrode ist ein Dielektrikum vorgesehen. Die elektrische Entladung erfolgt an der inneren Elektrode, so dass sich ein Plasma ausbildet.

Das deutsche Patent DE 10 2004 049 783 B4 offenbart eine Vorrichtung zur Bearbeitung von Gütern und unter Zuhilfenahme einer elektrischen Entladung. Auch hier ist eine Aufnahmekammer ausgebildet, in die der zu behandelnde Gegenstand eingebracht wird. An der Außenseite der Kammer sind wenigstens zwei Elektroden vorgesehen, die kapazitiv mit einer inneren Elektrode gekoppelt sind. Das Plasma wird in der Kammer generiert.

Die deutsche Patentanmeldung DE 10 2010 003 284 A1 offenbart ein Verfahren zur chemischen Aktivierung von Arbeitsgasen in abgeschlossenen Volumina. Die dielektrisch behinderte Entladung wird im Innern des Volumens erzeugt. Die felderzeugenden Elektroden haben dabei einen engen oberflächlichen äußeren Kontakt zum dielektrischen volumenbegrenzenden Material.

Die internationale Patentanmeldung WO 2011/055113 A1 beschreibt die Erzeugung eines Plasmas und die Verwendung der Plasmaerzeugungsvorrichtung. Die Vorrichtung zur Plasmaerzeugung benutzt eine erste Elektrode und eine zweite Elektrode. Die zweite Elektrode weißt eine Vielzahl von Spalten auf und bildet somit eine einheitliche Topologie des gebildeten Plasmas.

Die europäische Patentanmeldung EP 2 170 022 A1 offenbart einen Applikator für Plasma, um ein nicht-thermisches Plasma auf eine Oberfläche aufzubringen. Das Plasma dient zur Behandlung von lebendem Gewebe und im Besonderen zur Behandlung von Wunden. Hierzu ist eine Abdeckung vorgesehen, mit der die zu behandelnde Oberfläche abgedeckt werden kann und sich somit eine Kavität ausbildet. In die Kavität kann das nicht-thermische Plasma eingebracht werden, das mit der Oberfläche in Kontakt gelangt.

Der Erfindung liegt die Aufgabe zugrunde, eine Anordnung zur Keimreduktion mittels Plasma zu schaffen, die kostengünstig und einfach anzuwenden ist, ohne dass ein größerer apparativer und sicherheitstechnischer Aufwand notwendig ist. Hinzu kommt dass eine flächige und homogene Behandlung eines Bereichs erreicht werden soll.

Die obige Aufgabe wird mit einer Anordnung gemäß den Merkmalen des Anspruchs 1 gelöst.

Die erfindungsgemäße Anordnung zur Keimreduktion mittels Plasma umfasst einen piezoelektrischen Transformator. Der piezoelektrische Transformator ist in einem Gehäuse angeordnet. Ein Hochspannungsende des piezoelektrischen Transformators ist zu einer Öffnung im Gehäuse hin ausgerichtet. Ferner ist eine dielektrische Folie vorgesehen, die mit einem umlaufenden Rand einen zu entkeimenden Bereich ein- bzw. umschließt. Durch die Verwendung der dielektrischen Folie wird eine Kavität ausgebildet. Ein Hochspannungsende des piezoelektrischen Transformators ist einer der Kavität abgewandten Seite der dielektrischen Folie zugewandt. Das Plasma wird dabei innerhalb der Kavität gezündet.

Der Vorteil der erfindungsgemäßen Anordnung ist, dass eine flächige und homogene Behandlung einer Oberfläche oder eines zu entkeimenden Bereichs möglich ist. Die Folie kann an die herrschenden Bedingungen angepasst werden, so dass die erfindungsgemäße Vorrichtung sehr variabel einsetzbar ist. Hinzu kommt, dass für die Plasmaerzeugung keine Anregungsspannung von mehreren Kilovolt notwendig ist.

Gemäß der erfindungsgemäßen Anordnung lässt sich das Plasma ohne aktive Gegenelektrode in der Kavität zünden, wobei das Plasma rein durch kapazitive Feldübertragung in der Kavität angeregt wird.

Die dielektrische Folie kann gemäß einer Ausführungsform in einem flächigen Bereich durch eine elektrisch leitfähige Schicht verstärkt sein. Die elektrisch leitfähige Schicht verteilt das elektrische Feld in der durch die elektrisch leitfähige Schicht definierten Ebene, so dass eine Schädigung durch das Auftreten eines lokalen elektrischen Durchschlags verhindert ist. Die leitfähige Schicht ist dabei auf einen Teil der dem piezoelektrischen Transformator abgewandten Seite oder zugewandten Seite auf der dielektrischen Folie aufgebracht.

Der piezoelektrische Transformator ist dabei derart der dielektrischen Folie oder der leitfähigen Schicht der dielektrischen Folie zugeordnet, dass das Hochspannungsende des piezoelektrischen Transformators mechanisch und formschlüssig an der dielektrischen Folie oder der leitfähigen Schicht anliegt.

Ferner kann das Hochspannungsende des piezoelektrischen Transformators fest mit einer flächigen Elektrode verbunden sein, die aus dielektrischem und elektrisch leitfähigem Material besteht.

Der piezoelektrische Transformator kann zusammen mit einer Platine und Steuerschaltung zur Anregung des piezoelektrischen Transformators in einem Handgerät untergebracht sein. Das Handgerät selbst weist ein Gehäuse auf, wobei das Hochspannungsende des piezoelektrischen Transformators zu einer Öffnung im Gehäuse hin ausgerichtet ist.

Im Gehäuse selbst ist ein erster Druck vorgesehen, wobei der erste Druck kleiner ist als der Umgebungsdruck. Durch diese Bedingung wird erreicht, dass die dielektrische Folie an der Öffnung des Gehäuses angesaugt ist und bleibt. Somit ist sichergestellt, dass während der gesamten Anwendung des Niedertemperatur-Plasmas das Hochspannungsende des piezoelektrischen Transformators formschlüssig an der dielektrischen Folie anliegt. Zur Erzeugung des ersten Drucks im Gehäuse kann das Gehäuse mit einem Lüfterrad versehen sein, dass aus dem Inneren des Gehäuse die Luft absaugt und so den erforderlichen Unterdruck erzeugt.

Die Kavität, die den zu entkeimenden Bereich um- bzw. einschließt, wird dadurch gebildet, dass der umlaufende Rand der dielektrischen Folie mit dem Körper verklebt ist, der den zu entkeimenden Bereich trägt. Eine weitere Möglichkeit besteht darin, dass die Kavität zumindest teilweise mit einem porösen oder faserigen Material gefüllt ist, dass eine homogene Gasentladung in der Kavität unterstützt.

Gemäß einer weiteren Ausführungsform der Erfindung kann die Kavität durch eine dielektrische Folie gebildet sein, die den zu entkeimenden Gegenstand vollkommen umschließt. Die Kavität kann auch mit einem Prozessgas gefüllt sein.

Zur Energieversorgung des piezoelektrischen Transformators kann ein Akku oder ein Anschluss für ein Standardnetzteil vorgesehen sein. Die erfindungsgemäße Anordnung findet Verwendung bei der Keimreduktion bzw. Keimabtötung im Bereich des menschlichen oder tierischen Körpers. Hierzu ist der zu entkeimende Bereich innerhalb einer Kavität angeordnet, die dadurch gebildet wird, dass eine dielektrische Folie mit dem menschlichen oder tierischen Körper verbunden wird.

Ebenso findet die erfindungsgemäße Anordnung bei der Keimreduktion bzw. Keimabtötung von Lebensmitteln oder anderen hygienisch zu verpackenden Gegenständen Anwendung. Hierbei umschließt die dielektrische Folie die Lebensmittel oder die hygienisch zu verpackenden Gegenstände vollkommen.

Erfindungsgemäß werden mit dem piezoelektrischen Transformator elektrische Wechselfelder mit hoher lokaler Feldstärke erzeugt, wobei auch eine Gasentladung in einem Hohlraum gezündet werden kann, der durch eine dünne dielektrische Folie begrenzt ist. Am wirkungsvollsten geschieht diese Gasentladung, wenn die Hochspannungsseite des piezoelektrischen Transformators formschlüssig (spaltfrei) mit der dielektrischen Folie in Berührung gebracht wird. Zur leichteren und sicheren Handhabung, kann der piezoelektrische Transformator in einem Handgerät untergebracht sein, wobei das Handgerät für Batterie- oder Akkubetrieb ausgestaltet sein kann. Ebenso ist es möglich, dass das Handgerät mit einem Netzanschluss verbunden sein kann. Ferner hat es sich als vorteilhaft erwiesen, wenn in der Kavität eine poröse Struktur vorgesehen ist. In einer porösen Struktur zündet die Gasentladung besonders homogen, da in kleinen Poren die mittlere freie Weglänge, in der Gasphase bei Atmosphärendruck, vergleichbar mit der Porengröße (1 bis 10 µm) der porösen Struktur ist.

Weitere Vorteile und vorteilhafte Ausgestaltungen der Erfindung sind Gegenstand der nachfolgenden Figuren, sowie deren Beschreibungsteile.

Es zeigen im Einzelnen:
- **Figur 1**: eine schematische Ansicht des prinzipiellen Aufbaus des Handgeräts, das bei der gegenwärtigen Erfindung zur Erzeugung eines Plasmas bei Atmosphärendruck Anwendung findet;
- **Figur 2**: eine perspektivische Ansicht des Handgeräts von außen, bei der der piezoelektrische Transformator in einem Gehäuse untergebracht ist;
- **Figur 3**: eine Ausführungsform eines Handgeräts, das bei der erfindungsgemäßen Anordnung Verwendung findet;
- **Figur 4**: eine schematische Darstellung der Zuordnung des piezoelektrischen Transformators zu einer dielektrischen Folie, die die Kavität bildet;
- **Figur 5**: eine schematische Darstellung der Zuordnung des piezoelektrischen Transformators zu der dielektrischen Folie, wobei in der Kavität poröses Material vorgesehen ist;
- **Figur 6**: eine schematische Darstellung der Zuordnung des piezoelektrischen Transformators zu einer Kavität, wobei die dielektrische Folie einen zu entkeimenden Gegenstand vollkommen umschließt;
- **Figur 7**: eine schematische Ansicht des piezoelektrischen Transformators in Verbindung mit der dielektrischen Folie, wobei diese eine elektrisch leitfähige Schicht trägt; und
- **Figur 8**: eine schematische Ansicht des piezoelektrischen Transformators, der in einem Gehäuse angeordnet zur Plasmaerzeugung der dielektrischen Folie zugeordnet ist.

Für gleiche oder gleich wirkende Elemente der Erfindung werden identische Bezugszeichen verwendet. Die dargestellten Ausführungsformen stellen lediglich eine Möglichkeit dar, wie die Anordnung zur Keimreduzierung mittels eines Plasmas ausgestaltet sein kann.

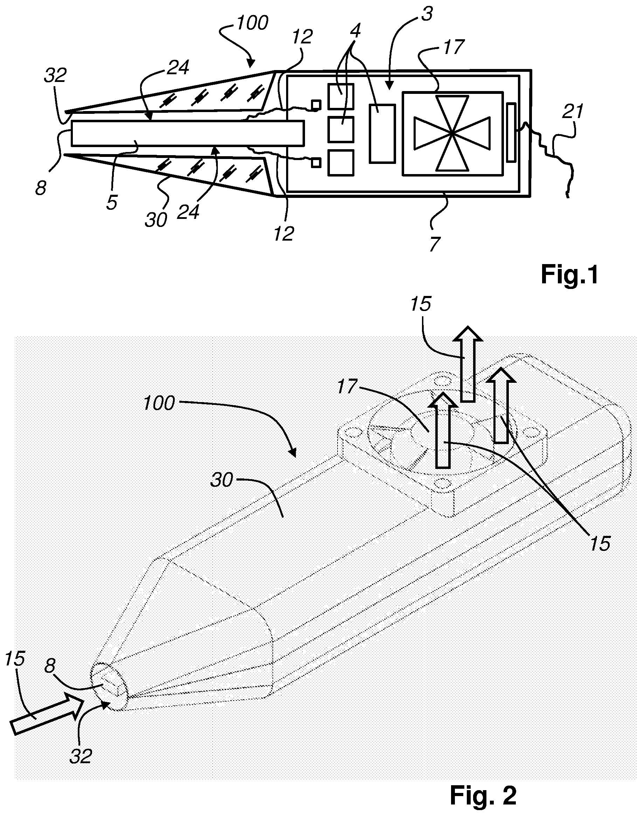

Eine schematische Draufsicht auf ein Handgerät 100, das bei der gegenwärtigen Erfindung Verwendung findet, ist in **Figur 1** dargestellt. Der piezoelektrische Transformator 5 ist in einem Gehäuse 30 untergebracht. Zur Ansteuerung ist der piezoelektrische Transformator 5 mit einer Platine 7 verbunden. Die Platine 7 realisiert mit einer Vielzahl von elektronischen Bauelementen 4 eine Steuerschaltung 3. Mit der Steuerschaltung 3 ist es möglich, den piezoelektrischen Transformator 5 mit seiner Resonanzfrequenz anzuregen. Die Steuerschaltung 3 für den piezoelektrischen Transformator 5 kann mit einer externen Energieversorgung verbunden werden, die ein herkömmliches Standard-Netzteil (nicht dargestellt) ist, das über ein Kabel 21 mit dem Gehäuse 30 des piezoelektrischen Transformators 5 verbunden ist. Ebenso kann die Energieversorgung mit einem Akku durchgeführt werden. Eine Kombination aus Akku und Standard-Netzteil ist ebenfalls denkbar. Die Ansteuerspannung wird von der Steuerschaltung 3 der Platine 7 über je einen elektrischen Anschluss 12 an je eine Seitenfläche 24 des piezoelektrischen Transformators 5 angelegt. Durch die an den Seitenflächen 24 des piezoelektrischen Transformators 5 anliegende Anregungsspannung bildet sich am Hochspannungsende 8 des piezoelektrischen Transformators 5 die erforderliche Hochspannung aus. Im oder an dem Gehäuse 30 ist ferner ein Lüfter 17 vorgesehen, der im Gehäuse 30 einen Druck erzeugt, der kleiner ist als der Umgebungsdruck. Dadurch ist es möglich, dass eine dielektrische Folie 6 ständig am Hochspannungsende 8 des piezoelektrischen Transformators 5 anliegt.

In **Figur 2** ist eine perspektivische Ansicht einer Ausführungsform des Gehäuses 30 dargestellt. In dem Gehäuse 30 ist der piezoelektrische Transformator 5 untergebracht. Das Hochspannungsende 8 des piezoelektrischen Transformators 5 ist über eine Öffnung 32 des Gehäuses 30 zugänglich. Der Lüfter 17, welcher mit dem Gehäuse verbunden ist, erzeugt einen Strom 15 des Umgebungsgases, so dass der Druck innerhalb des Gehäuses 30 kleiner ist, als der Umgebungsdruck. Damit wird erreicht, dass die dielektrische Folie 6 an die Öffnung 32 des Gehäuses 30 angesaugt wird. Somit ist auch sichergestellt, dass das Hochspannungsende 8 des piezoelektrischen Transformators 5 während des Gebrauchs des Handgeräts 100 ständig in Kontakt mit der dielektrischen Folie 6 ist.

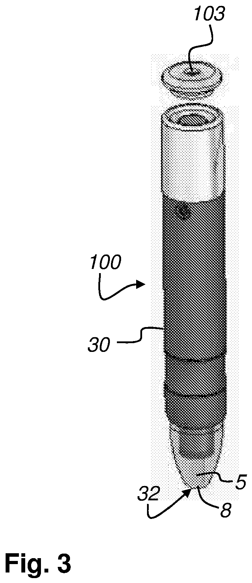

**Figur 3** zeigt eine Ausführungsform des Handgeräts 100, das für die Keimreduktion eines zu behandelnden Bereichs verwendet werden kann, der von der dielektrischen Folie 6 ein- bzw. umschlossen ist. Das Handgerät 100 ist mit einem Anschluss 103 für ein Kabel eines Standard-Netzteils ausgebildet. Das Handgerät 100 hat zylindrische Form, so dass im Innern des Gehäuses ebenfalls ein Lüfter (hier nicht dargestellt) angeordnet sein kann, der die in Figur 2 dargestellte Strömung 15 des Umgebungsgases erzeugt. Die Strömung 15 tritt durch die Öffnung 32 des Gehäuses 30 ein, so dass die dielektrische Folie 8 an der Öffnung 32 des Gehäuses 30 und am Hochspannungsende 8 des piezoelektrischen Transformators 5 anliegt.

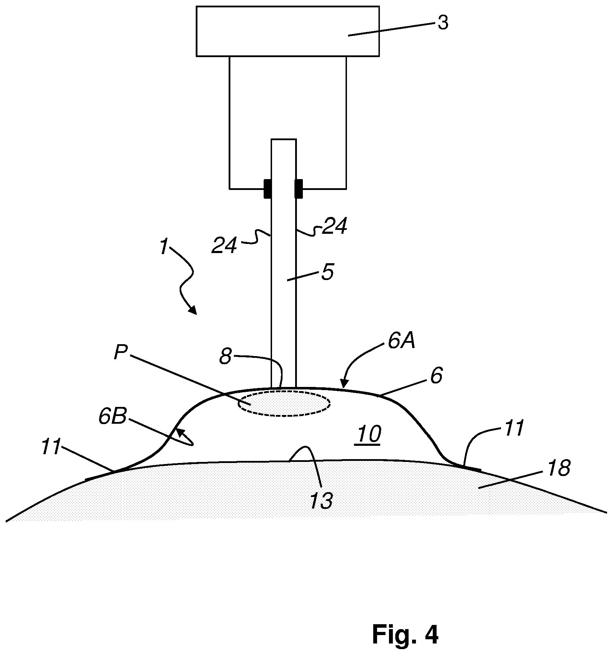

In **Figur 4** ist eine erfindungsgemäße Anordnung 1 schematisch dargestellt. Das Hochspannungsende 8 des piezoelektrischen Transformators 5 liegt an einer dielektrischen Folie 6 an. Die dielektrische Folie 6 besitzt einen umlaufenden Rand 11, der um einen zu entkeimenden Bereich 13 herum mit einem Körper 18 verklebt ist. Die dielektrische Folie 6 besitzt eine dem zu entkeimenden Bereich 13 abgewandte Seite 6A und eine zum entkeimenden Bereich zugewandte Seite 6B. Das Hochspannungsende 8 des piezoelektrischen Transformators 5 ist während der Anwendung mit der dem zu entkeimenden Bereich 13 abgewandten Seite 6A der Folie 6 in Kontakt. Der piezoelektrische Transformator 5 ist mit einer Steuerschaltung 3 verbunden. Hierzu ist die Steuerschaltung 3 elektrisch mit jeweils zwei gegenüberliegenden Seiten 24 des piezoelektrischen Transformators 5 elektrisch verbunden. Durch die Anregung 3 über die Steuerschaltung wird ein Plasma P (Gasentladung) erzeugt, dass sich innerhalb der Kavität 10 ausbildet. In anderen Worten, wird das Plasma P auf der dem zu entkeimenden Bereich 13 zugewandten Seite 6B der dielektrischen Folie 6 erzeugt.

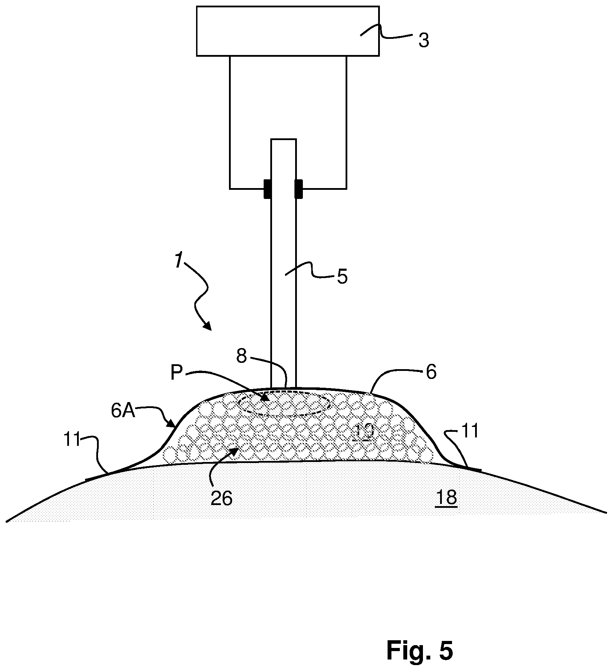

**Figur 5** zeigt eine weitere Ausführungsform der Anordnung 1, wobei das Hochspannungsende 8 des piezoelektrischen Transformators 5 ebenfalls der dem zu entkeimenden Bereich 13 abgewandten Seite 6A der dielektrischen Folie 6 zugeordnet ist. Wie bereits in der Beschreibung zu Figur 4 erwähnt, ist die dielektrische Folie 6 mit dem umlaufenden Rand 11 mit dem Körper 18 verklebt, so dass sich um den zu entkeimenden Bereich 13 die Kavität 10 ausbildet. In der hier dargestellten Ausführungsform ist die Kavität 10 mit einem porösen oder faserigen Material 26 gefüllt. Das poröse oder faserige Material 26 kann ebenfalls in Form eines Abstandskörpers ausgebildet sein, der auf den zu entkeimenden Bereich 13 gelegt wird und dann mit der dielektrischen Folie 6 derart umschlossen wird, dass der umlaufende Rand 11 der dielektrischen Folie 6 mit dem Körper 18 verklebt wird. Das poröse oder faserige Material 26 unterstützt dabei die Ausbildung des Plasmas P (Gasentladung) in der Kavität 10. Das poröse oder faserige Material 26 hat den Vorteil, dass hier die Gasentladung besonders homogen zündet. Die kleinen Poren im faserigen oder porösen Material 26 sind dabei derart ausgebildet, dass die mittlere freie Weglänge der Gasentladung in der Gasphase bei Atmosphärendruck vergleichbar der Porengröße (1 bis 10 µm) des Materials 26 ist.

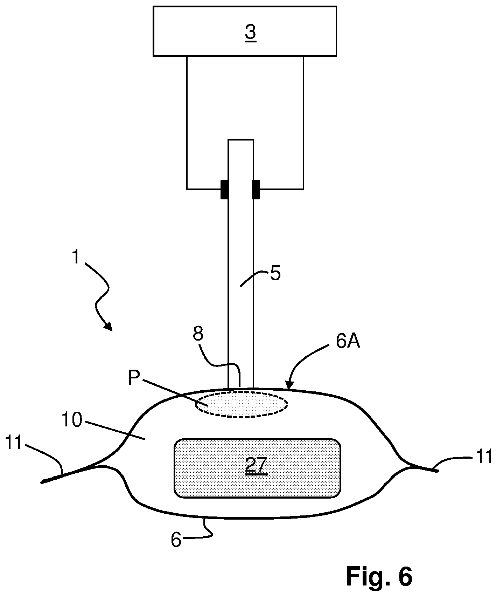

**Figur 6** beschreibt eine weitere Ausführungsform der Anordnung 1, wobei hier der zu entkeimende Gegenstand 27 komplett von der dielektrischen Folie 6 umgeben ist. Auch hier ist das Hochspannungsende 8 des piezoelektrischen Transformators 5 der der Kavität 10 abgewandten Seite 6A der dielektrischen Folie 6 zugewandt. Im Innern der Kavität 10 wird das Plasma P gezündet, um somit den Gegenstand 27 zu entkeimen. Die Kavität 10 kann beispielsweise dadurch gebildet werden, dass zwei dielektrische Folien 6 mit ihren umlaufenden Rändern 11 miteinander verklebt sind.

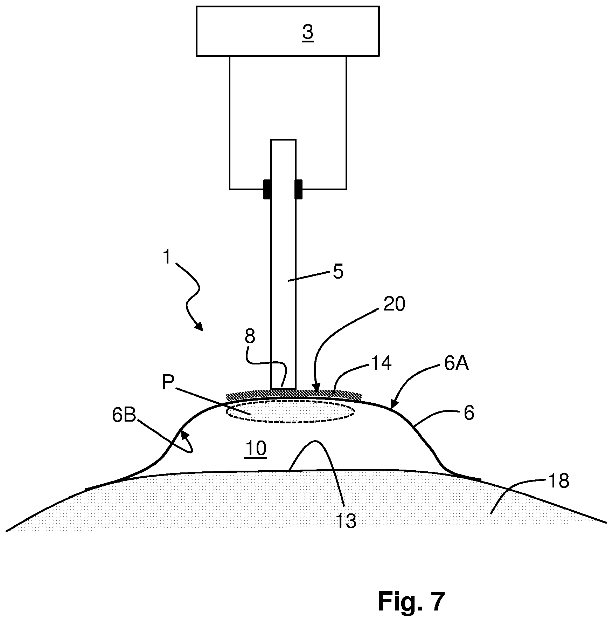

**Figur 7** zeigt eine weitere Ausführungsform der erfindungsgemäßen Anordnung 1. Um die Feldstärke optimal zu verteilen, ist eine leitfähige Schicht 14 auf die dielektrische Folie 6 aufgebracht. Die leitfähige Schicht 14 kann auf der der Kavität 10 abgewandten Seite 6A der dielektrischen Folie 6 oder auf der der Kavität 10 zugewandten Seite 6B der dielektrischen Folie 6 aufgebracht sein. Das Hochspannungsende 8 des piezoelektrischen Transformators 5 ist dabei in direktem Kontakt mit der leitfähigen Schicht 14, wenn sich diese auf der der Kavität 10 abgewandten Seite 6A der dielektrischen Folie 6 befindet. Anstatt der leitfähigen Schicht 14 kann das Hochspannungsende 8 des piezoelektrischen Transformators 5 mit einer flächigen Elektrode 20 versehen sein, die dann ihrerseits an der der Kavität 10 abgewandten Seite 6A der dielektrischen Folie 6 anliegt. Der zu entkeimende Bereich 13 wird, wie bereits in der Beschreibung zu den obigen Figuren erwähnt, durch die dielektrische Folie 6 festgelegt, die auf einen Körper 18 aufgebracht oder mit diesem verklebt ist.

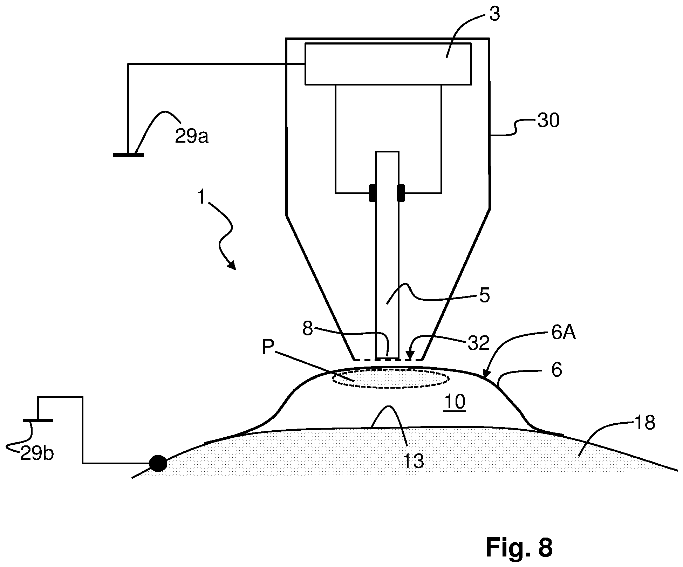

**Figur 8** zeigt eine Ausführungsform der erfindungsgemäßen Anordnung 1, bei der der piezoelektrische Transformator 5 in einem Gehäuse 30 angeordnet ist. Das Hochspannungsende 8 des piezoelektrischen Transformators 5 ist über die Öffnung 32 des Gehäuses 30 mit der der Kavität 10 abgewandten Seite 6A der dielektrischen Folie 6 in Kontakt. Um eine spaltfreie Ankopplung des Hochspannungsendes 8 des piezoelektrischen Transformators 5 zu erreichen, wird an das Gehäuse 30 ein Unterdruck angelegt, so dass die dielektrische Folie 6 an die Öffnung 32 des Gehäuses 30 angesaugt wird. Die in der Kavität 10 stattfindende Ausbildung des Plasmas P (Gasentladung) wird dadurch unterstützt, wenn die Steuerschaltung 3 für den piezoelektrischen Transformator 5 und der Körper 18, auf dem sich der zu entkeimende Bereich 13 befindet, an einer gemeinsamen Bezugsmasse 29a und 29b anliegen.

Die vorliegende Erfindung wird durch die folgenden Ansprüche definiert.

### Bezugszeichenliste:

- 1: Anordnung
- 3: Steuerschaltung
- 4: elektronische Bauelemente
- 5: piezoelektrischer Transformator
- 6: dielektrischen Folie
- 6A: der Kavität abgewandte Seite der Folie
- 6B: der Kavität zugewandte Seite der Folie
- 7: Platine
- 8: Hochspannungsende
- 10: Kavität
- 11: umlaufender Rand
- 12: elektrischer Anschluss
- 13: zu entkeimender Bereich
- 14: leitfähige Schicht
- 15: Strom des Umgebungsgases
- 17: Lüfter
- 18: Körper
- 20: flächige Elektrode
- 21: Kabel von Netzteil
- 24: Seite des piezoelektrischen Transformator
- 26: poröses oder faseriges Material
- 29a: Bezugsmasse
- 29b: Bezugsmasse
- 30: Gehäuse
- 32: Öffnung
- 100: Handgerät
- 101: Akku
- 103: Anschluss

## Patentansprüche

1. Anordnung (1) zur Keimreduktion mittels Plasma (P) umfassend:
einen piezoelektrischen Transformator (5);
eine Platine (7) mit einer Steuerschaltung (3) zur Anregung des piezoelektrischen Transformators (5), wobei der piezoelektrische Transformator (5) und die Platine (7) in einem Gehäuse (30) angeordnet sind; und
ein Hochspannungsende (8) des piezoelektrischen Transformators (5) zu einer Öffnung (32) im Gehäuse (30) hin ausgerichtet ist;
**dadurch gekennzeichnet,**
**dass** die Anordnung (1)
eine dielektrische Folie (6) mit einem umlaufenden Rand (11) umfasst, die einen zu
entkeimenden Bereich (13) ein- beziehungsweise umschließt, und eine Kavität (10) ausbildet; und dass
das Hochspannungsende (8) des piezoelektrischen Transformators (5) einer der Kavität (10) abgewandten Seite (6A) der dielektrischen Folie (6) zugewandt ist und das Plasma (P) innerhalb der Kavität (10) zündbar ist; und
ein Lüfterrad (17) vorgesehen ist, mit dem im Gehäuse (30) ein erster Druck erzeugbar ist, der größer ist als ein in der Umgebung vorliegender zweiter Druck, so dass an der Öffnung (32) des Gehäuses (30) die dielektrische Folie (6) ansaugbar ist und das Hochspannungsende (8) des piezoelektrischen Transformators (5) formschlüssig an der dielektrischen Folie (6) anliegt.

2. Anordnung (1) nach Anspruch 1, wobei auf einem Teil, der dem piezoelektrischen Transformator (5) abgewandten Seite (6A) oder zugewandten Seite (6B), der dielektrischen Folie (6) eine leitfähige Schicht (14) aufgebracht ist.

3. Anordnung (1) nach Anspruch 1 oder 2, wobei das Hochspannungsende (8) des piezoelektrischen Transformators (5) mechanisch und formschlüssig an der dielektrischen Folie (6) oder der leitfähigen Schicht (14) anliegt.

4. Anordnung (1) nach Anspruch 1, wobei das Hochspannungsende des piezoelektrischen Transformators (5) fest mit einer flächigen Elektrode (20) verbunden ist, die aus dielektrischem und elektrisch leitfähigem Material besteht.

5. Anordnung (1) nach den vorangehenden Ansprüchen, wobei das Gehäuse (30), das den piezoelektrischen Transformator (5) und die Platine (7) mit der Steuerschaltung (3) umschließt, ein Handgerät (100) bildet.

6. Anordnung (1) nach den vorangehenden Ansprüchen, wobei die Kavität (10) dadurch gebildet ist, dass der umlaufende Rand (11) der dielektrischen Folie (6) mit dem zu entkeimenden Bereich (13) eines Körpers (18) verklebt ist.

7. Anordnung (1) nach Anspruch 6, wobei die Kavität (10) zumindest teilweise mit einem porösen oder faserigen Material (26) gefüllt ist, das eine homogene Gasentladung unterstützt.

8. Anordnung (1) nach Anspruch 6 oder 7, wobei der Körper ein menschlicher oder tierischer Körper (18) ist und der zu entkeimende Bereich sich innerhalb der Kavität (10) befindet, die durch die mit dem menschlichen oder tierischen Körper (18) verbundene dielektrische Folie (6) gebildet ist.

9. Anordnung (1) nach den Ansprüchen 1 bis 5, wobei die Kavität (10) durch die dielektrische Folie (6) gebildet ist, die den zu entkeimenden Gegenstand (27) vollkommen umschließt.

10. Anordnung (1) nach den Ansprüchen 6 bis 9, wobei die Kavität (10) mit einem Prozessgas gefüllt ist.

11. Anordnung (1) nach den vorangehenden Ansprüchen, umfassend eine Energieversogung des piezoelektrischen Transformators (5), **wobei** die Energieversorgung des piezoelektrischen Transformators (5) ein Akku (101) und/oder ein Anschluss (103) für ein Standard-Netzteil ist.

12. Verwendung der Anordnung gemäß den Ansprüchen 1 bis 5 zur Keimreduktion bzw. Keimabtötung bei Lebensmitteln oder anderen hygienisch zu verpackenden Gütern, wobei die dielektrischen Folie (6) die Lebensmittel oder die hygienisch zu verpackenden Güter umschließt.

## Claims

1. An arrangement (1) for germ reduction by means of plasma (P) comprising:
a piezoelectric transformer (5);
a circuit board (7) having a control circuit (3) for exciting the piezoelectric transformer (5), wherein the piezoelectric transformer (5) and the circuit board (7) are arranged in a housing (30); and
a high-voltage end (8) of the piezoelectric transformer (5) is oriented toward an opening (32) in the housing (30);
**characterized in that**
the arrangement (1) comprises a dielectric film (6) having a peripheral edge (11), which encloses or surrounds a region (13) to be sterilized and forms a cavity (10); and
the high-voltage end (8) of the piezoelectric transformer (5) faces toward a side (6A) of the dielectric film (6) facing away from the cavity (10) and the plasma (P) can be ignited inside the cavity (10); and
a fan wheel (17) is provided, with which a first pressure can be generated in the housing (30), which is greater than a second pressure present in the surroundings, so that the dielectric film (6) can be suctioned against the opening (32) of the housing (30) and the high-voltage end (8) of the piezoelectric transformer (5) presses against the dielectric film (6) in a formfitting manner.

2. The arrangement (1) according to Claim 1, wherein a conductive layer (14) is applied to a part of the dielectric film (6), the side (6A) facing away from or the side (6B) facing toward the piezoelectric transformer (5).

3. The arrangement (1) according to Claim 1 or 2, wherein the high-voltage end (8) of the piezoelectric transformer (5) presses mechanically and in a formfitting manner against the dielectric film (6) or the conductive layer (14).

4. The arrangement (1) according to Claim 1, wherein the high-voltage end of the piezoelectric transformer (5) is fixedly connected to a planar electrode (20), which consists of dielectric and electrically-conductive material.

5. The arrangement (1) according to any one of the preceding claims, wherein the housing (30) which surrounds the piezoelectric transformer (5) and the circuit board (7) having the control circuit (3) forms a handheld device (100).

6. The arrangement (1) according to any one of the preceding claims, wherein the cavity (10) is formed in that the peripheral edge (11) of the dielectric film (6) is adhesively bonded to the region (13) to be sterilized of a body (18).

7. The arrangement (1) according to Claim 6, wherein the cavity (10) is at least partially filled with a porous or fibrous material (26), which supports a homogeneous gas discharge.

8. The arrangement (1) according to Claim 6 or 7, wherein the body is a human or animal body (18) and the region to be sterilized is located inside the cavity (10), which is formed by the dielectric film (6) connected to the human or animal body (18).

9. The arrangement (1) according to any one of Claims 1 to 5, wherein the cavity (10) is formed by the dielectric film (6), which completely surrounds the object (27) to be sterilized.

10. The arrangement (1) according to any one of Claims 6 to 9, wherein the cavity (10) is filled with a process gas.

11. The arrangement (1) according to any one of the preceding claims, comprising a power supply of the piezoelectric transformer (5), wherein the power supply of the piezoelectric transformer (5) is a rechargeable battery (101) and/or a connection (103) for a standard power supply unit.

12. A use of the arrangement according to any one of Claims 1 to 5 for germ reduction and/or sterilization in foods or other products to be hygienically packed, wherein the dielectric film (6) surrounds the food or the products to be hygienically packed.

## Revendications

1. Agencement (1) pour la réduction des germes au moyen d'un plasma (P), comprenant :
un transformateur piézoélectrique (5) ;
une carte de circuit imprimé (7) avec un circuit de commande (3) pour exciter le transformateur piézoélectrique (5), le transformateur piézoélectrique (5) et la carte de circuit imprimé (7) étant disposés dans un boîtier (30) ; et
une extrémité haute tension (8) du transformateur piézoélectrique (5) étant orientée vers une ouverture (32) dans le boîtier (30) ;
**caractérisé en ce que**
l'agencement (1) comprend un film diélectrique (6) avec un bord périphérique (11) qui entoure une zone à stériliser (13) et forme une cavité (10); et
que l'extrémité haute tension (8) du transformateur piézoélectrique (5) est tournée vers un côté (6A) du film diélectrique (6) opposé à la cavité (10) et le plasma (P) peut être amorcé à l'intérieur de la cavité (10) ; et
une roue de ventilateur (17) est prévue, avec laquelle une première pression peut être produite dans le boîtier (30), laquelle est supérieure à une deuxième pression présente dans l'environnement, de sorte que le film diélectrique (6) peut être aspiré au niveau de l'ouverture (32) du boîtier (30) et que l'extrémité haute tension (8) du transformateur piézoélectrique (5) est en contact par complémentarité de forme avec le film diélectrique (6).

2. Agencement (1) selon la revendication 1, dans lequel une couche conductrice (14) est appliquée sur une partie du côté (6A) opposé au transformateur piézoélectrique (5) ou du côté (6B) tourné vers le transformateur piézoélectrique (5) du film diélectrique (6).

3. Agencement (1) selon la revendication 1 ou 2, dans lequel l'extrémité haute tension (8) du transformateur piézoélectrique (5) est en contact mécanique et par complémentarité de forme avec le film diélectrique (6) ou la couche conductrice (14).

4. Agencement (1) selon la revendication 1, dans lequel l'extrémité haute tension du transformateur piézoélectrique (5) est reliée de manière fixe à une électrode plane (20) constituée d'un matériau diélectrique et électriquement conducteur.

5. Agencement (1) selon les revendications précédentes, dans lequel le boîtier (30) qui entoure le transformateur piézoélectrique (5) et la carte de circuit imprimé (7) avec le circuit de commande (3) forme un appareil portatif (100).

6. Agencement (1) selon les revendications précédentes, dans lequel la cavité (10) est formée en collant le bord périphérique (11) du film diélectrique (6) avec la zone à stériliser (13) d'un corps (18).

7. Agencement (1) selon la revendication 6, dans lequel la cavité (10) est au moins partiellement remplie d'un matériau poreux ou fibreux (26) qui favorise une décharge de gaz homogène.

8. Agencement (1) selon la revendication 6 ou 7, dans lequel le corps est un corps humain ou animal (18) et la zone à stériliser se trouve à l'intérieur de la cavité (10) formée par le film diélectrique (6) relié au corps humain ou animal (18).

9. Agencement (1) selon les revendications 1 à 5, dans lequel la cavité (10) est formée par le film diélectrique (6) qui entoure complètement l'objet à stériliser (27).

10. Agencement (1) selon les revendications 6 à 9, dans lequel la cavité (10) est remplie d'un gaz de processus.

11. Agencement (1) selon les revendications précédentes, comprenant une alimentation en énergie du transformateur piézoélectrique (5), dans lequel l'alimentation en énergie du transformateur piézoélectrique (5) est un accumulatuer (101) et/ou un raccord (103) pour un bloc d'alimentation standard.

12. Utilisation de l'agencement selon les revendications 1 à 5 pour la réduction ou la destruction des germes dans des denrées alimentaires ou d'autres marchandises à emballer de manière hygiénique, le film diélectrique (6) entourant les denrées alimentaires ou les marchandises à emballer de manière hygiénique.
